Europäisches Patentamt

European Patent Office

Office européen des brevets

(11) Publication number: **0 191 128**
A1

# EUROPEAN PATENT APPLICATION

(21) Application number: 85104740.7

(22) Date of filing: 19.04.85

(51) Int. Cl.⁴: **A 61 K 7/48,** A 61 K 9/00, A 61 K 31/47, A 61 K 31/055

(30) Priority: 14.02.85 US 701779

(71) Applicant: **TERAD international, Inc., 2555 Swaner Place, Ogden Utah 84401 (US)**

(43) Date of publication of application: **20.08.86 Bulletin 86/34**

(72) Inventor: **Adams, Teresa C., 2555 Swaner Place, Ogden Utah 84401 (US)**
Inventor: **Quintero, Bill, 1220 West Pescos Drive, Mesa Arizona 85202 (US)**

(84) Designated Contracting States: **AT BE CH DE FR GB IT LI LU NL SE**

(74) Representative: **Bauer, Wulf, Dr., Wolfgang-Müller-Strasse 12, D-5000 Köln 51 (Marienburg) (DE)**

(54) Topical preparations for human skin irratations.

(57) A topical preparation for the aleviation of human skin irritations, including chapping, reddening, and tenderness, contains 8 Hydroxy Quinoline Sulfate or Parachlorometaxylenol in a formulation of carrier ingredients. The topical formulations can be in wipe-on stick form, lotions, powders, aerosols, creams, ointments, jellies and oils. The formulations are useful in combatting diaper rash, foot rash, hand and face chapping and other forms of minor human skin irritations.

EP 0 191 128 A1

Applicant: TERAD International, Inc., Ogden, Utah 84401
U.S.A.

Title: Topical Preparations for Human Skin Irritations

---

This invention relates to topical preparations for aleviation of minor human skin irritations, including diaper rash.

Parents have long recognized the discomfort caused to babies by the skin irritations known as diaper rash. When the breakdown of urea absorbed by a baby's diaper occurs, irritating combinations of ammonia result which cause skin irritation and can lead to serious skin infections of the baby's bottom if left untreated.

Numerous attempts have been made to ameliorate the adverse effects of diaper rash and similar skin irritations. Liberal use of talcum powder and other agents, such as baby oils and the like have been used for generations to absorb the urea and ammonia compounds or coat the baby's bottom to prevent or lessen the effects of the rash. More recently, creams, lotions and oils containing other ingredients have been prepared to treat diaper rash. Prior art attempts include those found in U.S. Patents Nos. 3,935,862; 3,964,486; 4,117,187; and 4,385,632. These and other preparations commercially available have been unsuccessful in doing more than reducing the adverse influence of the ammonia by-products of urea or in protecting the baby's bottom from prolonged exposure to the effects of ammonium compounds on the baby's skin.

However, once skin irritations, such as chapping, reddening, tenderness and the like, begin to appear, these preparations do little more than attempt to isolate the baby's skin until the body's natural healing processes reverse the irritation of the skin and the naturally healthy skin condition is gradually achieved.

It is therefore an objective of this invention to provide a preparation to aid in the care of skin irritations, such as chapping, rashes, reddening, tenderness and the like.

It is an additional objective of the invention to provide a topical preparation in a variety of carriers to aid in caring for skin irritations.

These and other objectives which will become apparent are achieved with the invention described below.

A topical preparation for the alevation of human skin irritations, including chapping, reddening, and tenderness, contains 8 Hydroxy Quinoline Sulfate or Parachlorometaxylenol in a formulation of carrier ingredients. The topical formulations can be in wipe-on stick form, lotions, powders, aerosols, creams, ointments, jellies and oils. The formulations are useful in combatting diaper rash, foot rash, hand and face chapping and other forms of minor human skin irritations.

A number of formulations of the invention have been made which can be employed to aid in the care of minor human skin irritations. Several examples of preferred embodiments of the invention follow to illustrate the range of formulations and the types of preparations available within the scope of the invention.

EXAMPLE No. 1

**DIAPER RASH CONTROL STICK**

| Contents | Percentage |
|---|---|
| Mineral Oil | 51.60 |
| Polyethylene | 15.00 |
| Petrolatum Jelly | 15.00 |
| Talc | 12.00 |
| Lanolin | 3.00 |
| Allantoin | 1.00 |
| Kaolin | 1.00 |
| Titanium Dioxide | 1.00 |
| 8 Hydroxy Quinoline Sulfate | .30 |
| Iron Oxides | .05 |
| Fragrance | .05 |

EXAMPLE No. 2

**DIAPER RASH CONTROL STICK**

| Contents | Percentage |
|---|---|
| Mineral Oil | 51.60 |
| Polyethylene | 15.00 |
| Petrolatum Jelly | 15.00 |
| Talc | 12.00 |
| Lanolin | 3.00 |
| Allantoin | 1.00 |
| Kaolin | 1.00 |
| Titanium Dioxide | 1.00 |

| | |
|---|---|
| Parachlorometaxylenol | .30 |
| Iron Oxides | .05 |
| Fragrance | .05 |

EXAMPLE No. 3

**DIAPER RASH LOTION**

| Contents | Percentage |
|---|---|
| Water | 68.59 |
| Aloe | 7.00 |
| Propylene Glycol | 2.67 |
| Bee Pollen | 1.50 |
| Collagen | .30 |
| TEA | .90 |
| Stearic Acid | 4.00 |
| Glyceryl Stearate | 3.50 |
| IPM | 5.00 |
| Cetyl Alcohol | .80 |
| JoJoBa Oil | .30 |
| Ergocalciferol | .10 |
| Tocopherol | .10 |
| Macademia Oil | .10 |
| Herbs Tea | 4.00 |
| Germaben II | .83 |
| Fragrance | .01 |
| 8 Hydroxy Quinoline Sulfate | .30 |

EXAMPLE No. 4

**DIAPER RASH LOTION**

| Contents | Percentage |
|----------|-----------:|
| Water | 68.59 |
| Aloe | 7.00 |
| Propylene Glycol | 2.67 |
| Bee Pollen | 1.50 |
| Collagen | .30 |
| TEA | .90 |
| Stearic Acid | 4.00 |
| Glyceryl Stearate | 3.50 |
| IPM | 5.00 |
| Cetyl Alcohol | .80 |
| JoJoBa Oil | .30 |
| Ergocalciferol | .10 |
| Tocopherol | .10 |
| Macademia Oil | .10 |
| Herbs Tea | 4.00 |
| Germaben II | .83 |
| Fragrance | .01 |
| Parachlorometaxylenol | .30 |

EXAMPLE No. 5

**DIAPER RASH AEROSOL TYPE**

| Contents | Percentage |
|----------|-----------:|
| Water | 68.59 |
| Aloe | 7.00 |
| Propylene Glycol | 2.67 |
| Bee Pollen | 1.50 |

- 6 -

| Contents | Percentage |
|---|---|
| Collagen | .30 |
| TEA | .90 |
| Stearic Acid | 4.00 |
| Glyceryl Stearate | 3.50 |
| IPM | 5.00 |
| Cetyl Alcohol | .80 |
| JoJoBa Oil | .30 |
| Ergocalciferol | .10 |
| Tocopherol | .10 |
| Macademia Oil | .10 |
| Herbs Tea | 4.00 |
| Germaben II | .83 |
| Fragrance | .01 |
| 8 Hydroxy Quinoline Sulfate | .30 |

EXAMPLE No. 6

**DIAPER RASH AEROSOL TYPE**

| Contents | Percentage |
|---|---|
| Water | 68.59 |
| Aloe | 7.00 |
| Propylene Glycol | 2.67 |
| Bee Pollen | 1.50 |
| Collagen | .30 |
| TEA | .90 |
| Stearic Acid | 4.00 |
| Glyceryl Stearate | 3.50 |
| IPM | 5.00 |
| Cetyl Alcohol | .80 |
| JoJoBa Oil | .30 |
| Ergocalciferol | .10 |
| Tocopherol | .10 |

- 7 -

0191128

| Mecademia Oil | .10 |
| Herbs Tea | 4.00 |
| Germaben II | .83 |
| Fragrance | .01 |
| Parachlorometaxylenol | .30 |

EXAMPLE No. 7

**CREAM**

| Contents | Percentage |
| --- | --- |
| Stearic Acid | 4.00 |
| GMS | 8.00 |
| Prop. Parobeu | .10 |
| Mineral Oil | 10.00 |
| Wickenol 163 | 2.00 |
| Solvlan 98 | 2.40 |
| Acetulan | 1.60 |
| Vitamin E | .80 |
| Avacado Oil | 2.00 |
| Selione | .80 |
| Germall-115 | .30 |
| Methylparabeu | .20 |
| Allantoin | .20 |
| P Glycol | 4.00 |
| Dowicil 200 | .05 |
| Aloe | 1.00 |
| Water | 61.45 |
| TEA | .80 |
| 8 Hydroxy Quinoline Sulfate | .30 |

EXAMPLE No. 8

**CREAM**

| Contents | Percentage |
|---|---|
| Steaeric Acid | 4.00 |
| GMS | 8.00 |
| Prop. Parobeu | .10 |
| Mineral Oil | 10.00 |
| Wickenol 163 | 2.00 |
| Solvlan 98 | 2.40 |
| Acetulan | 1.60 |
| Vitamin E | .80 |
| Avacado Oil | 2.00 |
| Selione | .80 |
| Germall-115 | .30 |
| Methylparobeu | .20 |
| Allantoin | .20 |
| P Glycol | 4.00 |
| Dowicil 200 | .05 |
| Aloe | 1.00 |
| Water | 61.45 |
| TEA | .80 |
| Parachlorometaxylenol | .30 |

EXAMPLE No. 9

**OINTMENT**

| Contents | Percentage |
|---|---|
| Petrolatum | 75.00 |
| Lanolin | 24.70 |
| 8 Hydroxy Quinoline Sulfate | .30 |

EXAMPLE No. 10

**OINTMENT**

| Contents | Percentage |
|---|---|
| Petrolatum | 75.00 |
| Lanolin | 24.70 |
| Parachlorometaxylenol | .30 |

EXAMPLE No. 11

**JELLY**

| Contents | Percentage |
|---|---|
| Water | 94.03 |
| Aloe Powder | .25 |
| Prop. Glycol | 3.00 |
| Carbopal · | .80 |
| TEA | .90 |
| Dowicil 200 | .05 |
| Germaben II | .67 |
| 8 Hydroxy Quinoline Sulfate | .30 |

EXAMPLE No. 12

**JELLY**

| Contents | Percentage |
|---|---|
| Water | 94.03 |
| Aloe Powder | .25 |
| Prop. Glycol | 3.00 |
| Carbopal | .80 |
| TEA | .90 |

| | |
|---|---:|
| Dowicil 200 | .05 |
| Germaben II | .67 |
| Parachlorometaxylenol | .30 |

EXAMPLE No. 13

**POWDER**

| Contents | Percentage |
|---|---:|
| Talc | 99.00 |
| Methylparaben | .20 |
| Propylparaben | .20 |
| Allantoin | .30 |
| 8 Hydroxy Quinoline Sulfate | .30 |

EXAMPLE No. 14

**POWDER**

| Contents | Percentage |
|---|---:|
| Talc | 99.00 |
| Methylparaben | .20 |
| Propylparaben | .20 |
| Allantoin | .30 |
| Parachlorometaxylenol | .30 |

EXAMPLE No. 15

**OIL**

| Contents | Percentage |
|---|---:|
| Mineral Oil | 98.00 |
| Lanolin | 1.65 |

| 8 Hydroxy Quinoline Sulfate | .30 |
| Fragrance | .05 |

EXAMPLE No. 16

## OIL

| Contents | Percentage |
| --- | --- |
| Mineral Oil | 98.00 |
| Lanolin | 1.65 |
| Parachlorometaxylenol | .30 |
| Fragrance | .05 |

While this invention has been described in a preferred embodiment, it will be understood that there are substantial equivalents which fall within the scope of the appended claims.

While as can be seen from the above examples a percentage of 0.30 of either 8 Hydroxy Quinoline Sulfate or Parachlorometaxylenol is preferred in all topical formulations, the percentage may vary between e.g. 0.1 and 0.5 and may be even higher as 0.5, e.g. 0.8. If a combination of 8 Hydroxy Quinoline Sulfate and Parachlorometaxylenol is used, the total content of both should preferrably be within the said limits.

Applicant: TERAD International, Inc., Ogden, Utah 84401
U.S.A.

Title:     Topical Preparations for Human Skin Irritations


C  L  A  I  M  S


1.    Topical preparation for human skin irritations,
comprising in combination:
   - 8 Hydroxy Quinoline Sulfate; and/or Parachlorometaxylenol; and
   - a carrier for said sulfate and/or xylenol suitable
     for topical application.


2.    A preparation as set forth in Claim 1, wherein said
carrier ingredients comprise Mineral Oil, Polyethylene,
Petrolatum Jelly, Talc, Lanolin, Allantoin, Kaolin,
Titanium Dioxide, Iron Oxides and Fragrance.


3.    A preparation as set forth in Claim 1 or 2, wherein
said carrier ingredients comprise Water, Aloe, Propylene
Glycol, Bee Pollen, Collagen, TEA, Stearic Acid,
Glyceryl Stearate, IPM, Cetyl Alcohol, JoJoBa Oil,
Ergocalciferol, Tocopherol, Macademia Oil, Herbs Tea,
Germaben II, and Fragrance.


4.    A preparation as set forth in Claim 1, 2 or 3,
wherein said carrier ingredients comprise Stearic Acid,
GMS, Prop. Parobeu, Mineral Oil, Wickenol 163, Solvlan
98, Acetulan, Vitamin E, Avacado Oil, Selione, Germall-115, Methylparobeu, Allantoin, P Glycol, Dowicil 200, Aloe, Water, and TEA.

5. A preparation as set forth in one of the Claims 1 to 4, wherein said carrier ingredients comprise Pretrolatum, and Lanolin.

6. A preparation as set forth in one of the Claims 1 to 5, wherein said carrier ingredients comprise Water, Aloe Powder, Prop. Glycol, Carbopal, TEA, and Germaben II.

7. A preparation as set forth in one of the Claims 1 to 6, wherein said carrier ingredients comprise Talc, Methylparaben, and Allantoin.

8. A preparation as set forth in one of the Claims 1 to 7, wherein said carrier ingredients comprise Mineral Oil, Lanolin and Fragrance.

9. A preparation as set forth in one of the Claims 1 to 8, wherein said carrier ingredients comprise Dowicil 200.

10. A preparation as set forth in one of the Claims 1 to 9, wherein said carrier ingredients comprise Propylparaben.

European Patent Office

**EUROPEAN SEARCH REPORT**

Application number

| | DOCUMENTS CONSIDERED TO BE RELEVANT | | EP 85104740.7 |
|---|---|---|---|

| Category | Citation of document with indication, where appropriate, of relevant passages | Relevant to claim | CLASSIFICATION OF THE APPLICATION (Int. Cl.4) |
|---|---|---|---|
| X | <u>DE - A - 1 617 484</u> (GAF CORP.)<br><br>  * Example 23; page 3, lines 11-21; page 22, line 23 - page 23, line 22 * | 1,2,5, 8 | A 61 K 7/48<br>A 61 K 9/00<br>A 61 K 31/47<br>A 61 K 31/055 |
| X | <u>US - A - 4 463 016</u> (N.L. BURGESS)<br><br>  * Abstract; examples * | 1,10 | |
| X | H. JANISTYN, "Handbuch der Kosmetika und Riechstoffe" 3rd edition, vol. 1, "Die kosmetischen Grundstoffe" 1978<br><br>DR. ALFRED HÜTHIG VERLAG, Heidelberg<br>pages 188, 483<br><br>  * Page 188 p-Chlormetaxylenol; page 483 8-Hydroxychinolinsulfat * | 1 | |
| X | G.A. NOVAK "Die kosmetischen Präparate" 3rd edition, vol. 2, "Rezeptur Rohstoffe wissenschaftliche Grundlagen", 1984<br><br>VERLAG FÜR CHEM. INDUSTRIE H. ZIOLKOWSKY KG, Augsburg<br>pages 970-978<br><br>  * Pages 972-978 * | 1-10 | **TECHNICAL FIELDS SEARCHED (Int. Cl.4)**<br>A 61 K 7/00<br>A 61 K 9/00<br>A 61 K 31/00 |

The present search report has been drawn up for all claims

| Place of search | Date of completion of the search | Examiner |
|---|---|---|
| VIENNA | 21-05-1986 | IRMLER |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82

0191128
Application number

## EUROPEAN SEARCH REPORT

**European Patent Office**

| | DOCUMENTS CONSIDERED TO BE RELEVANT | | EP 85104740.7 |
|---|---|---|---|

| Category | Citation of document with indication, where appropriate, of relevant passages | Relevant to claim | CLASSIFICATION OF THE APPLICATION (Int. Cl.4) |
|---|---|---|---|
| X | "HANDBOOK OF NON-PRESCRIPTION DRUGS" 5th edition, Pub. American Pharmaceutical Association, 1977 pages 317-323<br><br>* Pages 321-323 * | 1 | |
| A | DD - A - 123 640 (G. SYDOW)<br><br>* Claim 1 * | 1 | |
| A | GB - A - 2 076 286 (QUINODERM LTD.)<br><br>* Example * | 1 | |
| A | GB - A - 1 539 771 (QUINODERM LTD.)<br><br>* Example 1 * | 1 | |
| A | US - A - 4 485 091 (H. FITTON)<br><br>* Example 1 * | 1 | TECHNICAL FIELDS SEARCHED (Int. Cl.4) |
| A | US - A - 3 904 754 (J.V. MORELLE)<br><br>* Example 9 * | 1 | |
| A | H. JANISTYN, "Handbuch der Kosmetika und Riechstoffe" 2nd edition, vol. 3, "Die Körperpflegemittel" 1973<br><br>DR. ALFRED HÜTHIG VERLAG, Heidelberg pages 494-499 | 1-10 | |

The present search report has been drawn up for all claims

| Place of search<br>VIENNA | Date of completion of the search<br>21-05-1986 | Examiner<br>IRMLER |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82